(19) Europäisches Patentamt
European Patent Office
Office européen des brevets

(11) EP 1 345 687 B1

(12) EUROPEAN PATENT SPECIFICATION

(45) Date of publication and mention of the grant of the patent:
**26.06.2013 Bulletin 2013/26**

(21) Application number: **01998401.2**

(22) Date of filing: **28.11.2001**

(51) Int Cl.:
***B01J 20/04*** *(2006.01)* ***B01D 15/00*** *(2006.01)*
***A61M 1/36*** *(2006.01)*

(86) International application number:
**PCT/US2001/044660**

(87) International publication number:
**WO 2002/043859 (06.06.2002 Gazette 2002/23)**

(54) **CARTRIDGES USEFUL IN CLEANING DIALYSIS SOLUTIONS**

BEI DER REINIGUNGVONDIALYSELÖSUNGEN NÜTZLICHE PATRONEN

CARTOUCHES UTILES DANS LE NETTOYAGE DE SOLUTIONS DE DIALYSE

(84) Designated Contracting States:
**AT BE CH CY DE DK ES FI FR GB GR IE IT LI LU MC NL PT SE TR**

(30) Priority: **28.11.2000 US 723396**

(43) Date of publication of application:
**24.09.2003 Bulletin 2003/39**

(73) Proprietor: **Renal Solutions, Inc.**
**West Lafayette, IN 47906 (US)**

(72) Inventor: **WONG, Raymond, J.**
**Norman, OK 73072 (US)**

(74) Representative: **Grünecker, Kinkeldey, Stockmair & Schwanhäusser**
**Leopoldstrasse 4**
**80802 München (DE)**

(56) References cited:
**FR-A- 2 585 251** **GB-A- 1 467 880**
**US-A- 3 669 880** **US-A- 4 360 507**

- **DATABASE WPI Section Ch, Week 198417 Derwent Publications Ltd., London, GB; Class B06, AN 1984-104208 XP002207709 & JP 59 046964 A (DAIICHI KIGENSO KAGAKU K), 16 March 1984 (1984-03-16)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 345 687 B1

## Description

The present invention relates to cartridges such as ion exchange cartridges or adsorption cartridges which are useful, for instance, in dialysis. In particular, the present invention relates in general to the regeneration or purification of used dialysate fluids. The present invention further relates to methods of conducting dialysis using certain cartridges and also relates to methods of making the cartridges.

Dialysis is a treatment that removes the waste products and excess fluid that accumulate in the blood as a result of kidney failure. Chronic renal failure is when the renal function has deteriorated to about 25% of normal. This amount of deterioration causes significant changes in the blood chemistry and is about the time that people feel poorly enough that they seek medical care. If medical treatment is sought at that time, progression can be slowed. Late stage chronic renal failure is when kidney function has decreased to 15%. End stage renal failure is when kidney function is at 5% of normal. Death will most likely result without treatment at this point. As of 1998, there were 430,000 patients in the United States diagnosed with chronic renal failure, wherein the average life expectancy of a chronic renal failure patient is 2 ½ years. Some do live 20 years or more. Also, there are approximately as many patients yearly with acute renal failure as with chronic renal failure, approximately ½ of these patients need treatment. On the whole, acute patients are sicker and less stable than chronic patients. They are frequently in ICU or CCU and can't be moved. Acute patients die, recover kidney function, or go on to become chronic dialysis patients. There is no current cure for renal disease. However, one treatment is transplantation, which is where a human kidney is surgically placed in the body and connected to the bladder. Daily medication is needed to keep the body from rejecting the transplanted kidney. Also, there is peritoneal dialysis (PD). With this treatment, a mild saltwater solution containing dextrose and electrolytes called dialysate is put into the peritoneal cavity. Because there is a rich blood supply to this abdominal cavity, urea and other toxins from the blood and fluid are moved into the dialysate, thereby cleaning the blood. The dialysate is then drained from the peritoneum. Later "fresh" dialysate is again put into the peritoneum.

Also, there is hemodialysis. This is a method of blood purification in which blood is continually removed from the body and passed through a dialyzer (artificial kidney) where metabolic waste and excess water are removed and pH and acid/base balance are normalized. The blood is simultaneously returned to the body. The dialyzer is a small disposable device consisting of a semi-permeable membrane. The membrane allows the wastes, electrolytes, and water to cross but restricts the passage of large molecular weight proteins and blood cells. Blood is pumped across one side of the membrane as dialysate is pumped in the opposite direction across the other side of the membrane. The dialysate is highly purified water with salts and electrolytes added. The machine is a control unit which acts to pump and control pressures, temperatures, and electrolyte concentrations of the blood and the dialysate. The average length of one hemodialysis treatment is 3-5 hours.

There are several types of hemodialysis:

a) Single Pass - hemodialysis is the most common treatment for renal disease. Most hemodialysis treatments are performed with single pass dialysis machines. They are called single pass because the dialysate (cleaning solution) passes by the blood in the dialyzer one time and then is disposed. Single pass dialysis machines generally require:

1) a water source capable of delivering at least 1000-1500 ml/min (assuming a 50% rejection rate by the R.O. system)
2) a water purification system sufficient of providing a continuous flow of 500-800 ml/min of purified water.
3) an electrical circuit of at least 15 amps in order to pump and heal 500-800 ml of water/min.
4) a floor drain or any other receptacle capable of accommodating at least 500 ml of used dialysatelminute as well as the rejected water from the R.O. system.

b) Sorbent Dialysis

1) does not require a continuous water source, a separate water purification machine or a floor drain because it continuously regenerates a small volume of dialysate and incorporates a water treatment system within the machine. Therefore, sorbent systems are truly portable.
2) sorbent systems require only a 5 amp electrical source because they recycle the same small volume of dialysate throughout the dialysis procedure. The heavy duty dialysate pumps and heaters used for large volumes of dialysate in single pass dialysis are not needed.
3) the sorbent system can use 6 liters of tap water from which dialysate is made for an entire treatment.
4) the sorbent system uses a sorbent cartridge - which acts both as a water purifier and as a means to regenerate used dialysate into fresh dialysate. The infusate system acts with it to properly balance the electrolyte composition of the regenerated dialysate.

The sorbent cartridge containing zirconium phosphate (ZrP) and hydrous zirconium oxide (HZO) ion-exchange materials has been historically used for the REDY regeneration hemodialysis system. The scheme of the REDY cartridge is shown in Figure 1.

The principle of the REDY cartridge is based on the hydrolysis of urea to ammonium carbonate by the enzymatic reaction of urease. The ammonia and ammonium ions are then removed by the zirconium phosphate (NaHZrP) in exchange for the hydrogen ions and $Na^+$ ions, which are counter-ions in the cation exchanger. ZrP also serves as cation exchanger to remove Ca, Mg, K, and all toxic metals in dialysate, thus allowing to maintain a balance of electrolyte level in the patient's blood (Ca, Mg, K) by using an infusate system, as well as providing safety for dialysis treatment with regard to water quality. The carbonate from the urea hydrolysis then combines with the hydrogen ions in NaHZrP to form bicarbonate, which is delivered to the uremic patient as a base to correct for acidosis. The hydrous zirconium oxide (HZO) containing acetate as a counter ion serves as an anion exchanger to remove phosphate from uremic patients for the treatment of hyperphosphatemia. The material also prevents leaching of phosphate from NaHZrP and removes toxic anions (e.g., fluoride) in water that may cause harm to a patient during dialysis. The acetate released during ion exchange is also a base to correct for acidosis by acetate metabolism. The granular activated carbon in the cartridge is responsible for the removal of creatinine, uric acid, and nitrogenous metabolic waste of the patient as well as chlorine and chloramine from water. Thus the REDY regenerative dialysis system is efficient to provide both safety and simplicity of water treatment and hence convenience for hemodialysis. The efficacy and safety record of the system has been well established. Nevertheless, the REDY cartridge can produce a variation of dialysate composition and pH during the treatment with a continuous release of $Na^+$ by the cartridge. Thus the REDY dialysis therapy has to provide several dialysate prescriptions to balance the $Na^+$ level in the patient for the correction of hyper and hyponatremia. Also a conductivity alarm system is generally present to keep the $Na^+$ level in the dialysate below a safe limit with proper dilution. The $Na^+$ and bicarbonate level in the dialysate may vary with the BUN level of the patient.

In the area of peritoneal dialysis (PD), particular emphasis has to be put on (1) a minimum variation of dialysate composition and pH during the PD treatment and (2) cost and size of the cartridge. For example, the adsorption capacity requirement of sorbent PD may be lower than that of REDY cartridge. The variation of dialysate composition is particularly important since PD is a slow treatment with treatment duration up to 2-4 hours per day. Excessive donation of $Na^+$ by the cartridge to the patient should be avoided during the treatment. In order to control the release of $Na^+$, an understanding of the ion exchange mechanism of ZrP with ammonium ions and dialysate cations (Ca, Mg, K, and Na) is needed.

ZrP is an inorganic cation exchange material with the molecular structure as shown below:

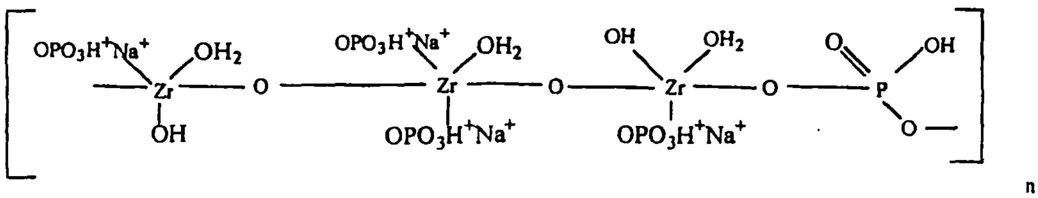

It contains both $H^+$ and $Na^+$ as counter-ions, which are responsible for ion exchange. The relative content of these ions in ZrP can be controlled by the pH to which acid ZrP (or $H^+$ZrP) is titrated with NaOH. The composition of the resultant product of titration, $Na_x^+H_{2-x}^+ZrP$, may vary during the following ion exchange processes in dialysate:

(i)

$$M^+ + \overline{Na^+H^+ZrP} \rightleftharpoons Na^+ + \overline{M^+H^+ZrP}$$

$$K = \frac{[Na^+]\,[\overline{M^+H^+ZrP}]}{[M^+]\,[\overline{Na^+H^+ZrP}]}$$

where $M^+ = NH_4^+$; $Ce^{2+}$; $Mg^{2+}$; $H^+$

(ii)

$$M^+ + \overline{Na^+H^+ZrP} \rightleftharpoons H^+ + \overline{M^+Na^+ZrP}$$

$$K = \frac{[H^+]\,[\overline{M^+Na^+ZrP}]}{[M^+]\,[\overline{Na^+H^+ZrP}]}$$

where $M^+ = Na^+$; $Ca^{2+}$; $Mg^{2+}$; $NH_4^+$
(iii)

$$Na_2CO_3 + \overline{Na^+H^+ZrP} \rightleftharpoons \overline{Na_2^+\,ZrP} + NaHCO_3$$

$$NaHCO_3 + \overline{Na^+H^+ZrP} \rightleftharpoons \overline{Na_2^+\,ZrP} + H_2CO_3 \longrightarrow H_2O + CO_2$$

The relative release of $N^{a+}$ and $H^+$ by ZrP during the ion exchange depends on the ion exchange equilibrium of these ions in ZrP with other cations in the liquid phase. The equilibrium may shift as the composition of $\overline{Na^+H^+ZrP}$ and liquid phase continue to change during the ion exchange process.

Based on the ion exchange principle, the $Na^+$ release from ZrP can be controlled by shifting to the conditions that favor the dominant release of $H^+$ ions. This concept can be important for the design of sorbent cartridge formulations for the PD fluid regeneration. The current method of making ZrP for the REDY cartridge is titrating acid ZrP ($H^+ZrP$) to the pH range 6.25 - 6.45 in a NaCl/NaAc buffer to produce $\overline{Na^+H^+ZrP}$ with high $Na^+$ content. This will trigger the $Na^+$ release especially in acetate or lactate dialysate with low buffer capacity and at low pH. Thus the ZrP quality made for the REDY cartridge may not be suitable for the PD fluid regeneration application. In order to remove this limitation, as shown in the present invention, a modification is made by using $\overline{Na^+H^+ZrP}$ with lower specified $Na^+$ content. This material can be made by titrating the acid ZrP ($H^+ZrP$) to a lower pH range 5.5 - 6.0 in deionized water. Another limitation of the REDY cartridge for PD treatment is that the hydrous zirconium oxide loaded with acetate is an acidic material. Thus the low pH of dialysate resulted from the acidity of this material will trigger a release of $Na^+$ and an initial loss of bicarbonate due to the reaction.

$$H^+ + HCO_3^- \rightleftharpoons H_2CO_3 \rightleftharpoons CO_2 \nearrow + H_2O$$

If the current REDY cartridge is used for PD treatment, it may produce a continuous rise of $Na^+$ concentration up to 170 mEq/l due to dominant $Na^+$ exchange throughout the treatment. In addition, an initial dip of $Na^+$. $HCO_3$ and pH may occur due to short time $H^+$ exchange.

Accordingly, in the area of dialysis, especially with respect to PD treatment, it would be beneficial to overcome one or more of the above-described disadvantages.

JP-A-59046964 discloses a blood clarifying material for direct haemo perfusion, which is prepared by encapsulating calcium substituted zirconyl carbonate with gelatin.

The problem underlying the present invention is to provide materials, which are useful in the regeneration or purification of solutions containing waste products; to provide materials which are useful in the regeneration or purification of dialysis solutions such as peritoneal dialysis solutions or other dialysis solutions such as those used in hemodialysis; to provide a system, wherein dialysis solutions can be regenerated in order to avoid large quantities of dialysis solutions and to avoid the discarding of spent dialysis solutions; and to over come one or more of the above described difficulties.

The problem is solved by a sorbent cartridge for purifying or regenerating dialysate fluids used in peritoneal dialysis (PD) or hemodialysis (HD) comprising sodium zirconium carbonate and zirconium phosphate, each of which is present as a lapper in said solvent cartridge.

Further, preferred embodiments are part of the subclaims.

The present invention also relates to a method of claim 6 and apparatuses of claims 7 and 8.

The apparatus can be a single pass dialysis system or a sorbent dialysis system. This apparatus with respect to a sorbent dialysis system further preferably contains an infusate pump, a dialyzer, a pump, and a reservoir all interconnected in an operating system as shown for instance in Figure 2.

Figure 1 is a schematic diagram showing a REDY® cartridge.
Figure 2 is a schematic diagram showing a single pass dialysis system.
Figure 3 is a schematic diagram showing a sorbent dialysis system.
Figures 4-6 are exploded views of preferred materials in sorbent cartridges of the present invention.
Figure 7 is a schematic diagram showing a dialysis test set-up to test the cartridges of the present invention.
Figure 8 is a diagram showing a cartridge and the various functions of each layer in a REDY® cartridge.

The present invention relates to materials useful for separation processes such as the removal of waste products and excess fluid that accumulates in dialysate fluids. These materials are present in a container (i.e., a cartridge) capable of holding the materials useful for the separation process. In a preferred embodiment, the materials described in detail below or the arrangement of various materials are preferably used in a dialysis system or other similar type of system that is useful for the removal of waste products and/or excess fluid that accumulates in dialysate fluids, for instance, as a result of conducting dialysis. As described in more detail below, the present invention is useful in purifying or regenerating dialysate fluids used in peritoneal dialysis (PD) and in hemodialysis (HD). For purposes of the present invention, a dialysis solution means a peritoneal dialysis solution or dialysate fluids that are useful in hemodialysis or sorbent dialysis systems. Conventional dialysis solutions for PD or HD can be used and regenerated by the present invention and are known to those skilled in the art.

The sodium zirconium carbonate preferably acts as a bicarbonate provider or donor and therefore other materials capable of acting as a bicarbonate provider or donor can also be used or alternatively can be used in the present invention. Other pH buffer materials that can be used as a bicarbonate provider in this invention include encapsulated sodium bicarbonate and ion exchange resins in carbonate form, and the like.

Preferably,

The sodium zirconium carbonate preferably has an average particle size of from 30 microns to 50 microns, and other particle size ranges can be used.

The sodium zirconium carbonate preferably, in its final form, has from 2 wt% to 5 wt% $Na^+$;
from 44 wt% to 50 wt% $ZrO_2$;
from 12 wt% to 18 wt% $CO_3^{2-}$; and
from 30 wt% to 40 wt% LOD, based on the weight of the sodium zirconium carbonate, wherein LOD is the amount of weight lost on drying of the SZC. The majority of the LOD will be $H_2O$.

The sodium zirconium carbonate preferably satisfies the standards set forth in ANSI/AAMI RD-5-1992 on extractable toxic impurities.

Preferably, the sodium zirconium carbonate achieves one or more of the following properties or characteristics:

- a phosphate adsorption having a minimum capacity of from 30 to 35 mg $PO_4$-P/gm SZC; other capacities can be used;
- a minimum $HCO_3^-$ content of from 2 to 4 mEq $HCO_3^-$/gm SZC; other amounts can be present;
- a maximum leachable $Na^+$ content of from 1.5 to 2.0 mEq $Na^+$gm SZC; other amounts can be present;
- and/or a pH range of the titrated sodium zirconium carbonate of from 6 to 7. Other pHs can be used.

Preferably, the sodium zirconium carbonate has at least one of the above characteristics and more preferably at least two or three, and even more preferably, all of the above characteristics.

The sodium zirconium carbonate preferably provides the necessary potency requirements for peritoneal dialysis or hemodialysis applications by providing a sufficient phosphate adsorption capacity for economic use as a clinical sorbent for the treatment of, for instance, hyperphosphatemia of renal disease patients. Further, the sodium zirconium carbonate of the present invention preferably provides the specified bicarbonate content in a peritoneal dialysis or hemodialysis fluid during applications. The present invention further has the minimum leachable Na+ as described above.

While any amount of the sodium zirconium carbonate can be used in the sorbent cartridge, preferably amounts effective to remove substantially all if not all of the phosphate in the waste material of the blood is present. For instance, amounts ranging from 50 grams to 250 grams of the sodium zirconium carbonate is present based on from 20 to 140 mEq$HCO_3^-$/L dialysate. More preferably, the amount of the sodium zirconium carbonate is from 80 to 120 grams. Other amounts above and below the above-recited ranges can be used. For purposes of the present invention, all amounts provided throughout as preferred amounts are based on 20 to 140 mEq$HCO_3^-$/L dialysate which is commonly used to determine the effectiveness of separation materials in the dialysis area. The examples of the present application further describe this simulated efficiency test.

Another component that can be optionally present in the sorbent cartridges of the present invention is an ammonia adsorbent such as, zeolite, titanium phosphate, zirconium silicate and organic ionic exchange resins. The zirconium phosphate (ZrP), is preferably titrated zirconium phosphate. Preferably the zirconium phosphate is titrated ZrP in the $Na^+$ and/or $H^+$ form. Preferably a mixture of $N^a$+ and $H^+$ are present in the ZrP.

More preferably, the zirconium phosphate has one or more of the following characteristics:

$H^+$ content of from 1.4 to 2.0 wt%;
$Na^+$ content of from 4 to 6 wt%;
$ZrO_2$ content of from 34 to 37 wt%;
$PO_4^-$ content of from 41 to 43 wt%; and
$H_2O$ content from 14 to 18 wt%, based on the weight of the zirconium phosphate. Other content amounts for the various characteristics can be used.

Furthermore, the zirconium phosphate used in the present invention preferably has an adsorption capacity for ammonia, $Ca^{2+}$, $Mg^{2+}$, $K^+$, and toxic heavy metals. More preferably, the adsorption capacity is approximately from 20 mg $NH_4$-N/gm ZrP to 45 mg or more $NH_4$-N/gm ZrP, and more preferably at least 30 mg $NH_4$-N/gm ZrP; from 2 mEq $Ca^{2+}$/gm ZrP to 7 mEq $Ca^{2+}$/gm ZrP, and more preferably at least 3 mEq $Ca^{2+}$/gm ZrP; from 1 mEq $Mg^{2+}$/gm ZrP to 5 mEq $Mg^{2+}$/gm ZrP, and more preferably at least 2 mEq $Mg^{2+}$/gm ZrP; and from 3 mEq HM/gm ZrP to 9 mEq HM/gm ZrP, and more preferably at least 6 mEq HM/gm ZrP for heavy metals (HM).

Further, the zirconium phosphate preferably has a $Na^+$ content of from 1.8 mEq $Na^+$/gm ZrP to 3 mEq $Na^+$/gm ZrP, and more preferably 2.4 mEq $Na^+$/gm and a pH of from 5.5 to 6. Other pHs can be used and different $Na^+$ contents can be used.

Also, the zirconium phosphate use in the present invention preferably has a minimum leachable $PO_4^{3-}$ for the material and more preferably is less than 0.05 mg $PO_4^{3-}$/gm ZrP. Other amounts can be used.

In addition, the zirconium phosphate preferably has an average grain size of from 30 to 40 microns and has no residual sulfate or chloride (e.g., less than 0.01%). Other particle sizes can be used. Furthertnore, the zirconium phosphate preferably satisfies the ANSI/AAMI RD-5-1992 standard on extractable toxic impurities and has a pH when in water of from 6 to 7.

The ammonia adsorbent, preferably zirconium phosphate, can be used in any amount. Preferably, the amount of the ammonia adsorbent is a sufficient amount to remove at least partially if not substantially or entirely all of the ammonia present in the spent fluids. More preferably, the amount of the ammonia adsorbent, and preferably zirconium phosphate, in a cartridge is from 300 grams to 750 grams and more preferably from 500 to 600 grams based generally on the dialysate mentioned above. This range is especially preferred for PD regeneration. Another range is preferably from 800 grams to 1900 grams in the cartridge and more preferably from 1300 grams to 1700 grams. These ranges are especially preferred by HD regeneration. Thus, overall ranges for the cartridge in general are from 300 grams or less to 1900 grams or more. Other amounts can be used.

Another component which can be present in the cartridges of the present invention is zirconium oxide and preferably hydrous zirconium oxide and more preferably hydrous zirconium oxide (HZO) containing acetate (HZO·Ac). The hydrous zirconium oxide containing acetate preferably acts as a counter ion and serves as an ion exchanger to remove phosphate from uremic patients. The hydrous zirconium oxide can also prevent leaching of phosphate from NaHZrP and removes toxic anions present in water that may cause harm to a patient during dialysis. The acetate released during ion exchange can also act as a base to correct for acidosis by acetate metabolism. The hydrous zirconium oxide described in the patents and publications below can be used herein. While the zirconium oxide and preferably hydrous zirconium oxide can be located anywhere in the cartridge, preferably in one embodiment, the hydrous zirconium oxide is adjacent or closer to the sodium zirconium carbonate. Even more preferably, and especially for HD regeneration systems, hydrous zirconium oxide is blended with the sodium zirconium carbonate. While any amount can be blended with the sodium zirconium carbonate, preferably a weight ratio at or near a 1 to 1 weight ratio is used. Thus, from 50 grams to 300 grams are preferred. A preferred weight ratio of hydrous zirconium oxide containing acetate and the sodium zirconium carbonate is an amount of from 50 grams/50 grams to 200 grams/200 grams and more preferably 100 grams/ 100 grams wherein the ratio signifies HZO·Ac to SZC. Other amounts can be used as well.

In addition, another component that can be present in the cartridges of the present invention is zirconium basic carbonate (ZBC) and/or other Group IV metal basic carbonates. Commercially available forms can be used as well as those described in any one of the patents and publications set forth below. A preferred Group IV metal carbonate is zirconium basic carbonate and more preferably has the following parameters:

a zirconium basic carbonate having $Na^+$ content of less than 1000 ppm;
a $ZrO_2$ wt% of from 35% to 40%; and
a $CO_3^{2-}$ wt% of from 8% to 10%, based on the weight of the zirconium basic carbonate. Other amounts for each of these parameters can be used.

Preferably, the zirconium basic carbonate has essentially no $SO_4^{2-}$ and essentially no $Cl^-$ in the zirconium basic carbonate, e.g., less than 0.01 wt%. The ZBC can be used in any amount and preferably from 100 grams to 600 grams in a cartridge.

Other materials that can also be present in the sorbent cartridge include, but are not limited to, alumina, alumina

supported urease, granulated activated carbon, activated alumina, zeolites, diatomaceous earth, direct urea sorbents, and other conventional adsorbent(s), fillers, and glass beads. The materials, amounts, and other optional components and/or dialysis systems described in the following patents and publications can also be used in the present application: US Design Patent No. 282,578, US-A-3,669,878, US-A-3,669,880, US-A-3,697,410, US-A-3,697,418, US-A-3,703,959, US-A-3,850,835, US-A-3,989,622, US-A-3,989,625, US-A-4,025,608, US-A-4,213,859, US-A-4,256,718, US-A-4,360,507, US-A-4,460,555, US-A-4,484,599, US-A-4,495,129, US-A-4,558,996; and the following articles, "Guide to Custom Dialysis," Product No. 306100-005, Revision E, pages 1-54, dated September 1993 and "Sorbent Dialysis Primer," Product No. 306100-006, Edition 4, pp. 1-51, dated September 1993 of Cobe Renal Care, Inc.

With respect to the sorbent cartridge, any combination of the above-described materials can be used.

The various combination of materials can be present as a mixture or present in any other type of arrangement. For instance, a series of cartridges can be used wherein the combination of the above-described materials can be present in one or more cartridges. For instance, the sodium zirconium carbonate can be present in one cartridge and the zirconium phosphate, for instance, can be present in a second cartridge, and optionally, the alumina is present in a third cartridge and so on. Alternatively, or in combination, one or more adsorbent cartridges can contain one or more of the above-described materials in any combination. Thus, for instance, sodium zirconium carbonate, for instance, can be present in one or more cartridges and the zirconium phosphate, for instance, can be present in the same or different cartridges and so on. More preferably, all of the materials are in a single cartridge and even more preferably arranged as layers in the cartridge.

Any effective amounts of the above-described materials can be optionally present in the cartridges of the present invention. For instance, alumina can be present in an amount of from 100 grams or below to 500 grams or above and more preferably from 200 grams to 350 grams and more preferably 300 grams. A preferred particle size for the alumina is from 20 microns or less to 40 microns or more. The alumina is commercially available from sources like Alcoa. With respect to the immobilized urease, preferably the immobilized urease is present in an amount of from 100 grams to 300 grams and more preferably from grams to 250 grams. Preferably the enzyme (e.g., urease) is immobilized by being mixed with a filler or the like such as alumina. A preferred source of urease is Jack Bean Meal commercially available from such sources as Sigma, preferably in the amount of from 8 or less to 20 grams or more. Generally, the urease is present in an amount of from 22,000 IU or less to 55,000 IU or more, and more preferably from 28,000 IU to 42,000 IU. The particle size of the Jack Bean Meal can be any effective size such as 40 mesh or less (or less than 0.4 mm). The activated carbon may be present in any amount and preferably is present in an amount of from 100 grams or less to 500 grams and more preferably from 200 grams to 300 grams and even more preferably at 250 grams. The activated carbon is available from such sources as Calgon. These optional materials are commercially available and various types are also described in the patents and publications identified herein. The particle sizes of the above materials are generally in the same ranges as for the SZC and/or ZrP, except for the activated carbon and immobilized enzyme. Preferred ranges are from 20 to 120 microns for alumina; and from 0.4 to 1.2 mm (or 12-50 mesh sieve) for the activated carbon.

Figures 4 through 6 provide exploded views of preferred materials in the adsorbent cartridges of the present invention as well as the preferred arrangement of the various layers contained in the adsorbent cartridges. Preferably, the flow of the spent dialysate fluid enters from the bottom of the cartridge and exits at the top of the cartridge which is more clearly shown in Figure 8, for instance. Figure 4 provides a more preferred cartridge set up for the processing of spent dialysate from PD fluids while Figures 5 and 6 provide a preferred set up for cartridges useful in the processing of spent dialysate from an HD system. Figure 5 is a more preferred set up for a cartridge useful for a 4 hour treatment while Figure 6 is a preferred set up for an 8 hour treatment as described herein. These Figures provide preferred amounts and material layer arrangement. However, as mentioned throughout this application, these various layers can be substituted with other suitable materials or the layers can be rearranged. As indicated in Figures 5 and 6, the layer containing the activated carbon or other suitable adsorbent can be located at the bottom of the cartridge and/or at the top of the cartridge.

The various amounts of the various ingredients described throughout this application can be present in effective amounts to accomplish the various functions of each ingredient as set forth, for instance, in Figure 8. Any combination is possible.

For purposes of the present invention, a sorbent cartridge is any container capable of being used for the separation or purification of a substance (which is preferably a liquid) and more preferably is used dialysate containing waste products and/or excess fluid. The sorbent cartridge generally has an inlet and an outlet and the cartridge can be any shape or size depending upon desired uses. Figure 1 provides one preferred design of a sorbent cartridge wherein the inlet and the outlet are identified as numeral 11 and numeral 13. Various shapes of the sorbent cartridge include, but are not limited to, a cylindrical shape, rectangular shape, a pyramidal-cylindrical shape as shown, for instance, in Figure 1 and so on. The shape can be straight-edged or tapered, and so on. Any geometric shape can generally be used.

Preferably, the PD cartridge has the following dimensions: 5,08 cm (2 inches), - 7,62 cm (3 inches) diameter by 12,7 cm (5 inches) to 25,4 cm (10 inches) length. The HD cartridge preferably has the following dimensions: 10,16 cm (4 inches) - 15,24 cm (6 inches) diameter by 15,24 cm (6 inches) - 30,48 cm (12 inches) long. Other dimensions can

be used depending on the needs of the purifying, amount to purify, operating system and the like. Examples of cartridges are also described in one or more of the patents and/or publications identified herein.

The above-described materials are arranged in layers preferably in one or more sorbent cartridges. In more detail, preferably, the sodium zirconium carbonate is present as a layer in the sorbent cartridge and the zirconium phosphate is present as a layer in the sorbent cartridge and preferably in the same sorbent cartridge. The layers of the various materials, if used, can be present in any order or combination. More preferably, the sodium zirconium carbonate is located as the layer closest to the outlet of the sorbent cartridge (i.e., top of cartridge in Figure 1). In other words, preferably, the used dialysate passes through other layers first, if present, and then passes through the sodium zirconium carbonate. A preferred arrangement of layers is shown in Figure 4. If an immobilized enzyme layer is present, preferably, this layer is located before the zirconium phosphate layer or other suitable layer, if present. Again, in other words, the used dialysate passes first through the immobilized enzyme layer prior to passing through the layer containing the zirconium phosphate, if present. Furthermore, while the sodium zirconium carbonate can be present any where in the cartridge, preferably the layer containing the sodium zirconium carbonate is located after the layer containing the zirconium phosphate, if present.

A preferred order of layers is as follows, realizing that these layers and the materials in the layers are optional.

a) sodium zirconium carbonate or other alkali metal-Group IV metal-carbonate
b) zirconium phosphate or other ammonia adsorbent
c) alumina or other like material
d) alumina supported urease or other immobilized enzyme layer or other material to convert urea to ammonia such as diatomaceous earth or zirconium oxide
e) granular activated carbon or other adsorbent.

Preferably, one or more filter pads can be located throughout the sorbent cartridge to ensure that the layer integrity is maintained during operation. The filter pad can be made of any type of material, for instance, standard filter paper or cellulose pads and the like and typically is the diameter or length-width of the cartridge in order to separate completely one layer from another layer. Preferably, a filter pad is located above and in contact with the sodium zirconium carbonate layer. A second filter pad can be located between and in contact with the alumina supported urease layer and the granular activated carbon layer. A third filter pad can be located between and in contact with the granular activated carbon layer that faces the inlet of the sorbent cartridge. One or more of these filter pads can be used. A flow diffuser which uniformly diffuses the used dialysate throughout the entire width or diameter of the sorbent cartridge can preferably be used. The flow diffuser preferably has a design of radial spreading channels made of plastic or other suitable materials. The flow diffuser is, as shown in Figure 4, typically located prior to any of the optional filter pads or materials used in the sorbent cartridge and is adjacent to the inlet (or part of the inlet) of the sorbent cartridge. A barrier layer(s) can also be used in the sorbent cartridge. A barrier layer is preferably located between the immobilized enzyme layer and the alumina layer, if present. An example of a barrier layer includes filter paper and the like.

In another embodiment of the present invention, the present invention relates to a particular order of materials present as layers in a sorbent cartridge as follows:

- adsorbent layer
- immobilized enzyme layer or other material to convert urea to ammonia
- optional barrier layer
- optional alumina layer
- ammonia adsorbent
- phosphate adsorbent
- optional filter pad

The particular arrangement of layers results in an optimal regeneration or purification of the used dialysate. In more detail, the following description of these materials and/or layers or other materials can be used in any combination in the present invention.

1. Immobilized urease or other enzyme layer for enzymatic conversion of urea to ammonium carbonate. The methods to immobilize urease may be categorized as follows:

a. Adsorption, e.g., alumina, activated carbon, anion-exchange resins, diatomaceous earth, or other conventional adsorbents that are commonly employed adsorbents.

b. Covalent bond to water insoluble polymer to form enzyme-polymer conjugates via activation procedure or reactive polymer. The commonly employed water-insoluble supports for the covalent attachment of enzymes may include the following:

| | |
|---|---|
| Synthetic supports, e.g., | - Acrylamide-based polymer |
| | - Maleic anhydride-based polymers |
| | - Polypeptides |
| | - Styrene-based polymer |
| Natural Supports, e.g., | - Agarose (sepharose) |
| | - Dextran (sephadex) |
| | - Cellulose |
| | - S tarch |

c. Intermolecular cross-linking of enzyme using multifunctional reagents e.g., glutaraldehyde, hexamethylene diamine. Cross-linking is usually after adsorption on porous support.
d. Entrapment within cross-linked polymers, e.g., polyacrylamide gel.
e. Microencapsulation, e.g. nylon, cellulose nitrate, ethyl cellulose, polyamide.
f. Containment within semi-permeable membrane devices, e.g., Amicon ultrafiltration cells, Dow hollow fiber beaker device.
2. Cation exchange materials in $Na^+$ or $H^+$ form as a $NH_4$-scavenger and adsorbent for infusate cations as well as toxic trace metals in tap water. Another function of the material is to convert carbonate from urea hydrolysis to bicarbonate. These may include cation exchange resins and inorganic ion exchange sorbents (cation type) such as zirconium phosphate, titanium phosphate, zeolite, and the like.
3. Anion exchange materials in $Ac^-$, $HCO_3^-$, $Cl^-$, or $OH^-$ form as a phosphate scavenger and adsorbent for toxic anions in tap water such as $F^-$ and aluminate. Another function of the materials can be to provide base supplement such as acetate and bicarbonate in order to correct for metabolic acidosis of the patient. These may include anion-exchange resins and inorganic ion-exchange sorbent (anion type) such as hydrous zirconium oxide, hydrous silica, stannic oxide, titanium oxide, antimonic acid, hydrous tungsten oxide, and sodium zirconium carbonate.
4. Adsorbent for removal of creatinine, uric acid, and middle molecules of uremic toxins as well as organics in tap water. Although activated carbon is the most effective sorbent for removing nitrogenous waste metabolites, other potential candidates may include certain ion-exchange resins and affinity chromatography materials such as derivatives of cellulose, polystrene gel, polyacrylamide gels, porous glass and agarose.
5. For hemodialysis, a component layer is preferred to remove the chlorine from tap water for dialysis or to use highly purified water. The material can be carbon-impregnated pads, granular activated charcoal, and the like.

In the sorbent cartridge, the component materials can be put in a mixed form or arranged in discrete layers separated by filter papers or cellulose pads, although the efficiency and performance are different. There can be various configurations for the layers. The chlorine removal layer, if used, preferably precedes the urease-immobilized layer since chlorine can deactivate the enzyme. The $NH_4$- scavenger or cation exchange layer preferably succeeds the urease-immobilized layer.

The cartridges of the present invention, as indicated above, can be used in a variety of separation systems and preferably are used in the regeneration or purification of dialysates (e.g., HD) or PD solutions. In the most simplest design, spent or used dialysate or PD solutions can simply be passed through one or more cartridges to purify or regenerate the spent fluids. Such a system can be quite simple in setup and can involve merely using a column-type setup wherein the spent fluids are passed from top to bottom wherein gravity permits the spent fluid to go through the cartridge or spent fluid can be passed through the cartridge under pressure which permits the spent fluids to be introduced in any direction, for instance as shown in Figure 1. In a more elaborate system, the system set forth in Figure 2 can be used especially for hemodialysis; that is a single pass dialysis system or a system that is preferably used as a closed system as shown in Figure 3. With respect to the system shown in Figure 2, in lieu of discarding the used dialysate to a floor drain, as an alternative, the used dialysis can simply be collected in a container which then can be regenerated or purified by passing the spent dialysate through one or more cartridges as described above. More preferably, the sorbent dialysis system shown in Figure 3 uses a cartridge as described above which is located as indicated in Figure 3. Such a system permits the continuous reusing of the regenerated dialysate in a patient during dialysis treatment.

With respect to peritoneal dialysis, there are several options. First, like hemodialysis, the peritoneal dialysis solution that is spent can be directly passed through one or more cartridges to purify or regenerate the used peritoneal dialysis solution in order to remove the waste products. Alternatively, the peritoneal dialysis solution which is used or spent can first be passed through a dialyzer in the same manner as blood during hemodialysis wherein dialysate removes waste products and the like from the peritoneal dialysis solution and then the dialysate can be regenerated or purified by passing the used or spent dialysate through the cartridge. Either system can be used in the present invention. With

a closed PD system, such as one like Figure 3, the risk of peritonitis can be reduced significantly since the frequent connections which must be made with conventional systems between the catheter in the peritoneal cavity and a succession of dialysis solution containers is avoided in one embodiment of the present invention.

In more detail, and referring to Figure 2, in a single pass dialysis system, 15 identifies a source for electricity to operate the single pass dialysis system. 17 represents a flow meter, 19 represents a conductivity meter, 21 represents a temperature meter, and 23 represents a heater, all of which are conventional items used in single pass dialysis systems and are known to those skilled in the art and can be used in the system of the present invention. 25 represents a blood leak detector and 27 represents a UF meter which again are conventional items in a single pass dialysis system that are understood by those skilled in the art. 29 represents a dialyzer which again is known by those skilled in the art and typically is a system containing a membrane in order to have the waste products pass through the membrane to the dialysate fluid. There are a variety of different dialyzers commercially available and any of these can be used in the present invention. 31 represents the passing of the used dialysis and 33 represents the introduction of fresh dialysate into the dialyzer 29. 35 represents a UF control which is known to those skilled in the art in dialysis systems and conventional units can be used in the present invention. 37 represents a UF pump and 39 represents a proportioning pump which are conventional items in dialysis systems. 41 represents concentrate used to form the fresh dialysate, 43 represents the water used to mix with the concentrate in order to form the fresh dialysate and 45 represents the water treatment system used to purify the water prior to the mixing of the water with the concentrate. Again, these items are conventional in dialysis systems and commercially available items can be used in the present invention. 47 represents a container or drain to collect the used dialysate in order to be purified or regenerated by the cartridges of the present invention.

Referring to Figure 3, 49 refers to a source of electricity to operate the dialysis system shown in Figure 3. 51 represents a heater, 53 represents a flow meter, 55 represents a conductivity meter, 57 represents a temperature meter, and 59 represents a UF control. These items are conventional items in a sorbent dialysis system and are known to those skilled in the art and can be used in the present invention as shown in Figure 3. 61 is an infusate pump that is used to pump in fresh concentrate 79 to be mixed with the regenerated dialysate which ultimately enters the reservoir 77 which is preferably a six liter reservoir. 63 represents a blood leak detector and 65 represents a UF meter which are conventional items in dialysis systems and can be used herein. 67 represents a dialyzer which is the same as in Figure 2. Similarly, 69 represents used dialysis leaving the dialyzer and 71 represents fresh dialysate entering the dialyzer 67. 73 is a pump to pump the used dialysate from the dialyzer into the cartridge 75 which are the cartridges of the present application.

In a preferred embodiment, the cartridges of the present invention are made for 4 hours of dialysis treatment or for 8 hours of dialysis treatment. Furthermore, the 8 hour cartridges are typically made for home use and the 4 hour cartridges are typically made for dialysis treatment in medical treatment or dialysis centers.

The cartridges of the present invention can generally be used with any type of dialysis system as described above. The flows that pass through the cartridge are typically any conventional flows. For instance, flows from about 50 ml/min or less to 500 ml/min or more of dialysate can flow through the cartridge and can be used in the systems of the present invention. Other flows can be used depending upon the size of the cartridge and the operating system.

The cartridges of the present invention have the ability to maintain and/or restore in dialysate the $Na^+$ and/or $HCO_3^-$ amounts that should be present in fresh dialysate as well as in a patient's blood that is being treated by way of the dialysis system of the present invention. Accordingly, the present invention can restore the $Na^+$ and/or $HCO_3^-$ levels in spent dialysate to proper and acceptable levels, (e.g., for $Na^+$, from 135 to 145 mEq/L and for $HCO_3^-$, from 24 mEq to 32 mEq/L). As a result, restoring these levels permits the patient's blood to be restored and/or maintained at these levels. This is an impressive capability.

The dialysis systems or components thereof described in the following patents can be used in the present application and these systems can incorporate the materials and/or cartridges of the present invention: U.S. Patent Nos. 6,309,673; 6,306,836; 6,196,992; 6117,122; 6,074,359; 6,017,942; 5,984,891; 5,955,450; 5,938,634; 5,782,796; 5,631,025; 5,597,805; 4,560,472; 6,299,769; 6,284,131; 6,146,536; 5,968,966; 5,704,915; 5,824,213; 5,641,405; 4,738,668; 6,293,921; 6,284,139; 6,274,103; 5,980,481; and 5,498,338.

There are numerous uses for the materials of the present invention and especially the cartridges of the present invention such as the regeneration of dialysis fluids as mentioned above. Furthermore, the cartridges can also be used in any separation process which requires the removal of impurities or waste products from a fluid or other medium that is passable through the materials of the present invention. Also, the present invention is quite useful with respect to treating drug overdose patients or other patients which are in need or removing undesirable or dangerous contaminants in a person's system. Accordingly, the present invention provides useful embodiments that allow the regeneration of dialysate type fluids and other fluids.

The present invention will be further clarified by the following examples, which are intended to be purely exemplary of the present invention.

## EXAMPLES

The dialysate regeneration system was set up as shown in Figure 7. As shown in Figure 7, a test system can be arranged wherein there are various locations where the sampling of the dialysate fluid can occur as well as sampling of the simulated patient fluid which can be the simulation of the waste products found in blood or a PD fluid. In more detail, Sampling No. I provides the ability to sample the content of the spent dialysate fluid or PD fluid prior to its entry into the cartridge. Sampling No. 2 permits the testing of the dialysate or PD fluid once it has been regenerated by the cartridge of the present invention. Sampling No. 3 permits the sampling of the regenerated dialysate fluid or PD fluid after the fluid has been infused with the standard components of an infusate which typically introduces calcium and magnesium ions so as to restore them to acceptable levels. Other components can also be restored by the infusate. Sampling No. 4 permits the testing of the contents of the simulated fluids of a patient after passing through the dialyzer and Sampling No. 5 permits the ability to test the contents of a simulated patient's fluid containing general waste products typically found in blood or PD fluid or the like. The remaining components as shown in Figure 7 are conventional with respect to the mechanical set up and the use of sample ports, flow meters, flow controls, pumps, power controls, dialyzers, and the like.

### Example 1 - Cartridge for HD Regeneration

In this model, the patient was represented by a simulated fluid bath of 60 liters volume of the following composition at 37°C.

| | |
|---|---|
| $NaHCO_3$ | 25 mEq/L |
| NaCl | 115 mEq/l |
| $CaAc_2 \cdot H_2O$ | 3 mEq/L |
| $MgAc_2 \cdot 4H_2O$ | 1 mEq/L |
| KAc (anhydrous) | 2 mEq/L |
| Dextrose | 100 mg% |
| pH | 7-7.4 |
| Uremic Toxins Levels: | |
| BUN | ~85 mg% |
| Creatinine | ~9mg% |
| $PO_4$-P | ~6 mg% |

The simulated patient was dialyzed by using a Baxter PSN-120 dialyzer at the blood flow rate (BFR) of 180 ml/min. The 6L dialysate was made up in tap water with a starting concentration as follows:

| | |
|---|---|
| $NaHCO_3$ | 140 mEq/L |
| NaCl | 0 mEq/l |
| Dextrose | 100 mg% |
| pH | 8.1 |

In one experiment, the spent dialysate was regenerated by a sorbent cartridge of the following configuration at the dialysate flow rate (DFR) of 250 **ml/min.**

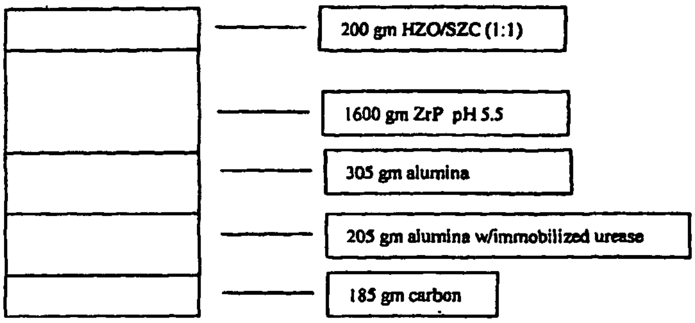


Infusate was provided at a calibrated flow rate to maintain $Ca^{2+}$, $Mg^{2+}$, and $K^+$ balance in the regenerated dialysate. The efficacy and performance of the treatment are demonstrated by the following results:

Uremic Toxin Level

Efficacy

| | | | |
|---|---|---|---|
| Pre-dialysis BUN level | 85 mg% | Post-dialysis BUN level | 36.6 mg% |
| Pre-dialysis creatinine level | 9.5 mg% | Post-dialysis creatinine level | 5.2 mg% |
| Pre-dialysis $PO_4$-P level | 4.7 mg% | Post-dialysis $PO_4$-P level | 2.8 mg% |

Amount of Uremic Toxin Removal from Patient and Adsorption Capacity of Cartridge

| | |
|---|---|
| BUN | 29 gm |
| Creatinine | 2.6 gm |
| $PO_4$-P | 1.16 gm |

These amounts of uremic toxin removal should meet the dialysis requirement based on kT/v.

Performance

Pressure generated by cartridge: 165 kPa (24 psi) max.

Composition of cartridge effluent during treatment:

| | |
|---|---|
| $NH_4$-N | 0 mg% |
| BUN | < 0.2 mg% |
| $Ca^{2+}$ | 0 mEq/L |
| $Mg^{2+}$ | 0.2 mEq/L |
| $K^+$ | < 0.3 mEq/L |
| $PO_4$-P | 0 mg% |
| Creatinine | < 0.8 mg% |

$Na^{\pm}$ and Bicarbonate Balance in Dialysate and Patient Fluid

The $Na^+$ level in cartridge outlet variation range was 132-155 mEq/L during treatment.

Pre-dialysis $Na^+$ of patient 136 mEq/l

(continued)

| | |
|---|---|
| Post-dialysis $Na^+$ of patient | 138 mEq/L |
| Pre-dialysis bicarbonate of patient | 24 mEq/L |
| Post-dialysis bicarbonate of patient | 25 mEq/L |

The above-described HD experiment was repeated using a variety of different parameters. The parameters that were varied are set forth in the Tables below. In each case, the cartridge having the various identified materials operated within acceptable parameters with respect to regenerating the dialysate.

| Test # | Dialysis Conditions | | | HZO/SZC | ZP Amt | ZP pH | NaHCO$_3$ Dialysate | Pre-Dialysis BUN | Patient Fluid Volume |
|---|---|---|---|---|---|---|---|---|---|
| | DFR/BFR | Dialyzer | k (ml/min) | gm | gm | | mEq/L | mg/DL | filters |
| 3 | 400/400 | Fresenius 7NR | 256 | 75/125 | 1300 | 5.5 | 40 | 87.5 | 40 |
| 4 | 400/400 | Fresenius 7NR | 256 | 100/100 | 1600 | 5.5 | 60 | 104.7 | 55 |
| 5 | 250/180 | PSN-120 | 125 | 140/60 | 1400 | 6 | 140 | 71.9 | 40 |
| 6 | 250/180 | PSN-120 | 125 | 100/100 | 1600 | 5.75 | 140 | 46 | 42 |
| 7 | 250/180 | PSN-120 | 200 | 100/100 | 1600 | 5.75 | 140 | 85 | 61 |

| Results | | | | | | |
|---|---|---|---|---|---|---|
| Test # | Treatment Time | Urea-N Hydrolyzed | $Na^+$ | | Bicarbonate | |
| | | | Post- | Pre- | Post- | Pre- |
| | min | gm | mEq/L | mEq/L | mEq/L | mEq/L |
| 3 | 173 | 23.4 | 144 | 137 | 26 | 25 |
| 4 | 235 | 38 | 142 | 139 | 26 | 21 |
| 5 | 420 | 20.71 | 142 | 135 | 31 | 24 |
| 6 | >480 | 14.7 | 141 | 138 | 20 | 25 |
| 7 | 420 | 29.5 | 138 | 136 | 26 | 24 |

Ave. $Na^+$ Donation per mEq Cation or $NH_4^+$ Adsorbed: ZrP pH 5.5, 0.106 mEq $Na^+$ ZrP pH 6.25, 0.2266 mEq $Na^+$

| Test # | ZrP Amt | ZrP pH | Total Infusate Cations & $NH_4^+$ Ions Adsorbed by ZrP | | |
|---|---|---|---|---|---|
| | | | $Ca^{2+}$, $Mg^{2+}$, $K^+$ | $NH_4^+$ | Total |
| | (grams) | | (mEq) | (mEq) | (mEq) |
| 3 | 1300 | 5.5 | 415 | 1671 | 2086 |
| 4 | 1600 | 5.5 | 564 | 2714 | 3278 |
| 5 | 1400 | 6 | 630 | 1479 | 2109 |
| 6 | 1600 | 5.75 | 720 | 1050 | 1770 |
| 7 | 1600 | 5.75 | 630 | 2109 | 2739 |

| Test # | Patient $Na^+$ Balance | | | $Na^+$ Donation per mEq Cation / NH4 + Adsorbed |
|---|---|---|---|---|
| | Post- | Pre- | Gain | |
| | mEq/L | mEq/L | mEq/L | mEq $Na^+$ |
| 3 | 144 | 137 | +280 | 0.1342 |
| 4 | 142 | 139 | +165 | 0.0503 |
| 5 | 142 | 135 | 280 | 0.1328 |
| 6 | 141 | 138 | 126 | 0.0712 |
| 7 | 138 | 136 | 122 | 0.0445 |
| GAIN = (Post-dialysis $Na^+$ Level - Pre-dialysis $Na^+$ Level) x patient dialyzable fluid volume V | | | | |

Example 2- Cartridge for PD Fluid Regeneration

The two-loop PD regenerative system using a dialyzer was set up according to Figure 7. The spent PD fluid was represented by a simulation bath of the following composition:

| | |
|---|---|
| Dextrose | 100 mg% |
| $Na^+$ | 138 mEq/L |
| $Ca^{2+}$ | 2.5 mEq/L |
| $Mg^{2+}$ | 1.0 mEq/L |
| $Cl^-$ | 113 mEq/L |
| $HCO_3^-$ | 25 mEq/L |
| BUN | 20 mg% |
| Creatinine | 3.6 mg% |
| $PO_4$-P | 4.1 mg% |
| pH | 7.4 |

The spent PD fluid was contained in a 15L PD bag and dialyzed by a Baxter CA50 dialyzer while it is re-circulated by pump at the flow rate of 100 ml/min. Uremic toxins (urea, creatinine, and phosphate) were continuously added to the regenerated PD fluid to simulate peritoneal dialysis in action and to replace the amount removed by the regenerative dialysis. The dialysate used for the regenerative dialysis was a bicarbonate solution of the following composition contained in a 4L PD bag.

| | |
|---|---|
| Dextrose | 1.5% |
| $Na^+$ | 135 mEq/L |
| $Ca^{2+}$ | 2.9 mEq/L |
| $Mg^{2+}$ | 0.5 mEq/L |
| $Cl^-$ | 92 mEq/L |
| $HCO_3^-$ | 31 mEq/L |
| pH | 7.4 |

The dialysate was re-circulated through a sorbent cartridge for purification at the flow rate of 100 ml/min, and provided continuously with infusate after regeneration to replace the $Ca^{2+}$ and $Mg^{2+}$ removed by the cartridge, so that the $Ca^{2+}$ and $Mg^{2+}$ levels in the PD fluid can be maintained and controlled.

The sorbent cartridge used to purify the PD dialysate has the following configuration:

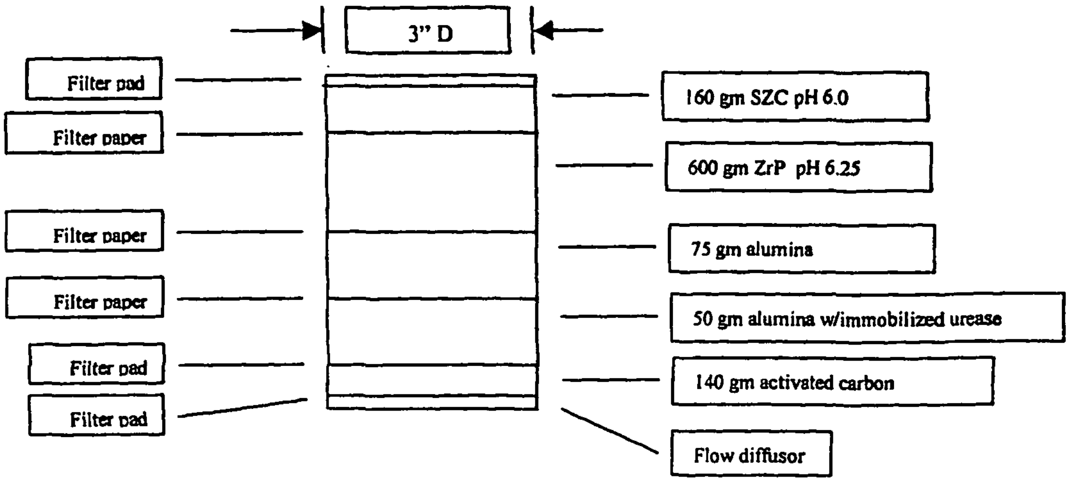


The cartridge served to continuously remove the uremic toxins (urea, creatinine, and phosphate) that were dialyzed across the dialyzer from the spent PD fluid into the dialysate. At the same time, it served to remove the $Ca^{2+}$ and $Mg^{2+}$ from the dialysate so that with the help of infusate provisions, the balance of these ions in the PD fluid was maintained.

The efficacy and performance of the cartridge can be summarized as follows:

Efficacy

The uremic toxin removal for an 8-hour treatment at 100 ml/min dialysate flow rate were as shown below:

| | |
|---|---|
| BUN | 4.8 gm |
| Creatinine | 0.96 gm |
| $PO_4$-P | 0.48 gm |

<u>Performance</u>

Electrolyte balance of PD fluid and dialysate before and after dialysis:

| | PD Fluid | | Dialysate | |
|---|---|---|---|---|
| | Pre-Dialysis | Post-Dialysis | Pre-Dialysis | Post-Dialysis |
| I $Na^+$ mEq/L | 141 | 137 | 135 | 145 |
| $HCO_3^-$ mEq/L | 26.5 | 25 | 31 | 33.5 |
| $Ca^{2+}$ mEq/L | 3.0 | 3.0 | 2.9 | 2.8 |
| $Mg^{2+}$ mEq/L | 1.0 | 1.0 | 1.0 | 1.0 |

Adsorption efficiency: No leakage of uremic toxic and $Ca^{2+}$, $Mg^{2+}$, were observed from the cartridge.

## Claims

1. A sorbent cartridge for purifiying or regenerating dialysate fluids used in peritoneal dialysis (PD) or in hemodialysis (HD) comprising sodium zirconium carbonate and zirconium phosphate, each of which is present as a layer in said sorbent cartridge.

2. The sorbent cartridge of claim 1, wherein said zirconium phosphate has an average grain size of from 30 to 40 μm

(microns).

3. The sorbent cartridge of claim 1, comprising layers have the following order:

a) sodium zirconium carbonate;
b) zirconium phosphate;
c) alumina;
d) alumina supported urease;
e) granular activated carbon.

4. The sorbent cartridge of claim 3, wherein said sorbent cartridge further comprises a first filter pad located above and in contact with said sodium zirconium carbonate, a second filter pad located between and in contact with said alumina supported urease and said granular activated carbon. and a third filter pad located heneath and is contact with said granular activated carbon.

5. The sorbent cartridge of claim 4, further comprising a flow diffuser located beneath and in contact with said third filter pad.

6. A method to regenerate or purify spent dialysis fluid comprising passing said spent dialysis fluid through the sorbent cartridge of claim 1

7. An apparatus for use in a dialysis treatment comprising the sorbent cartridge of claim 1 and a dialyzer in fluid communication with said cartridge, which allows spent dialysis fluid to pass from said dialyzer to and through said cartridge.

8. An apparatus for use in a dialysis treatment comprising the sorbent cartridge of claim 1 and a source of spent peritoneal dialysis solution, wherein the source of said spent peritoneal dialysis solution is in fluid communication with said cartridge, which allows the spent peritoneal dialysis solution to pass to and through that cartridge.

## Patentansprüche

1. Sorptionsmittelpatrone zum Reinigen oder Regenerieren von Dialysatfluids, welche bei der Peritonealdialyse (PD) oder bei der Hämodialyse (HD) verwendet werden, umfassend Natriumzirkoniumcarbonat und Zirkoniumphosphat, von denen jedes als eine Schicht in der Sorptionsmittelpatrone vorhanden ist.

2. Sorptionsmittelpatronen nach Anspruch 1, wobei das Zirkoniumphosphat eine durchschnittliche Korngröße von 30 bis 40 $\mu$m (Mikron) aufweist.

3. Sorptionsmittelpatrone nach Anspruch 1, umfassend Schichten in der folgenden Reihenfolge:

a) Natriumzirkoniumcarbonat;
b) Zirkioniumphosphat;
c) Aluminiumoxid;
d) Aluminiumoxid getragene Urease;
e) granulärer aktivierter Kohlenstoff.

4. Sorptionsmittelpatrone nach Anspruch 3, wobei die Sorptionsmittelpatrone des Weiteren ein erstes Filterpad umfasst, angeordnet oberhalb und in Kontakt mit dem Natriumzirkoniumcarbonat, ein zweites Filterpad angeordnet zwischen und in Kontakt mit der Aluminiumoxid getragenen Urease und dem granulären aktiviertem Kohlenstoff und ein drittes Filterpad angeordnet unter und in Kontakt mit dem granulären aktivierten Kohlenstoff.

5. Sorptionsmittelpatrone nach Anspruch 4, des Weiteren umfassend einen Flowdiffuser bzw. Strömungsdiffuser, welcher neben und in Kontakt mit dem dritten Filterpad angeordnet ist.

6. Verfahren zum Regenieren oder Reinigen von verbrauchtem Dialysefluid, umfassend das Durchführen des verbrauchten Dialysefluids durch die Sorptionsmittelpatrone nach Anspruch 1.

7. Vorrichtung zur Verwendung in einer Dialysebehandlung umfassend die Sorptionsmittelpatrone nach Anspruch 1 und einen Dialysator in Fluidverbindung mit der Patrone, welche es ermöglicht, das verbrauchte Dialysefluid von dem Dialysator zu und durch die Patrone zu leiten.

8. Vorrichtung zur Verwendung in einer Dialysebehandlung umfassend die Sorptionsmittelpatrone nach Anspruch 1 und eine Quelle verbrauchter peritonealer Dialyselösung, wobei sich die Quelle der verbrauchten peritonealen Dialyselösung in Fluidverbindung mit der Patrone befindet, welche es ermöglicht, das verbrauchte peritoneale Dialysefluid zu und durch die Patrone zu leiten.

## Revendications

1. Cartouche de sorbant permettant de purifier ou de régénérer des fluides de dialysat utilisés dans la dialyse péritonéale (PD) ou dans l'hémodialyse (HD) comprenant du carbonate de zirconium sodium et du phosphate de zirconium dont chacun est présent sous forme d'une couche dans ladite cartouche de sorbant.

2. Cartouche de sorbant selon la revendication 1, dans laquelle ledit phosphate de zirconium présente une taille moyenne de grain allant de 30 à 40 $\mu$m (microns).

3. Cartouche de sorbant selon la revendication 1, comprenant des couches qui présentent l'ordre suivant :

   a) carbonate de zirconium sodium,
   b) phosphate de zirconium,
   c) alumine,
   d) uréase supportée d'alumine,
   e) carbone granulaire activé.

4. Cartouche de sorbant selon la revendication 3, dans laquelle ladite cartouche de sorbant comprend en outre un premier coussin de filtrage situé au-dessus et en contact avec ledit carbonate de zirconium sodium, un second coussin de filtrage situé entre ladite uréase supportée d'alumine et ledit carbone granulaire activé, et en contact avec eux, ainsi qu'un troisième filtre situé en dessous et en contact avec ledit carbone granulaire activé.

5. Cartouche de sorbant selon la revendication 4, comprenant en outre un répartiteur d'écoulement situé en dessous et en contact avec ledit troisième coussin de filtre.

6. Procédé pour régénérer ou purifier un fluide de dialyse passé comprenant le passage dudit fluide de dialyse passé au travers de la cartouche de sorbant de la revendication 1.

7. Appareil à utiliser dans un traitement de dialyse comprenant la cartouche de sorbant conforme à la revendication 1 et un dialyseur en communication par fluide avec ladite cartouche, qui permet au fluide de dialyse passé de circuler depuis ledit dialyseur vers ladite cartouche et au travers de celle-ci.

8. Appareil à utiliser dans un traitement de dialyse comprenant la cartouche de sorbant conforme à la revendication 1 et une source de solution de dialyse péritonéale passée, dans lequel la source de ladite solution de dialyse péritonéale passée se trouve en communication par fluide avec ladite cartouche ce qui permet à la solution de dialyse péritonéale passée de circuler vers de cette cartouche et au travers de celle-ci.

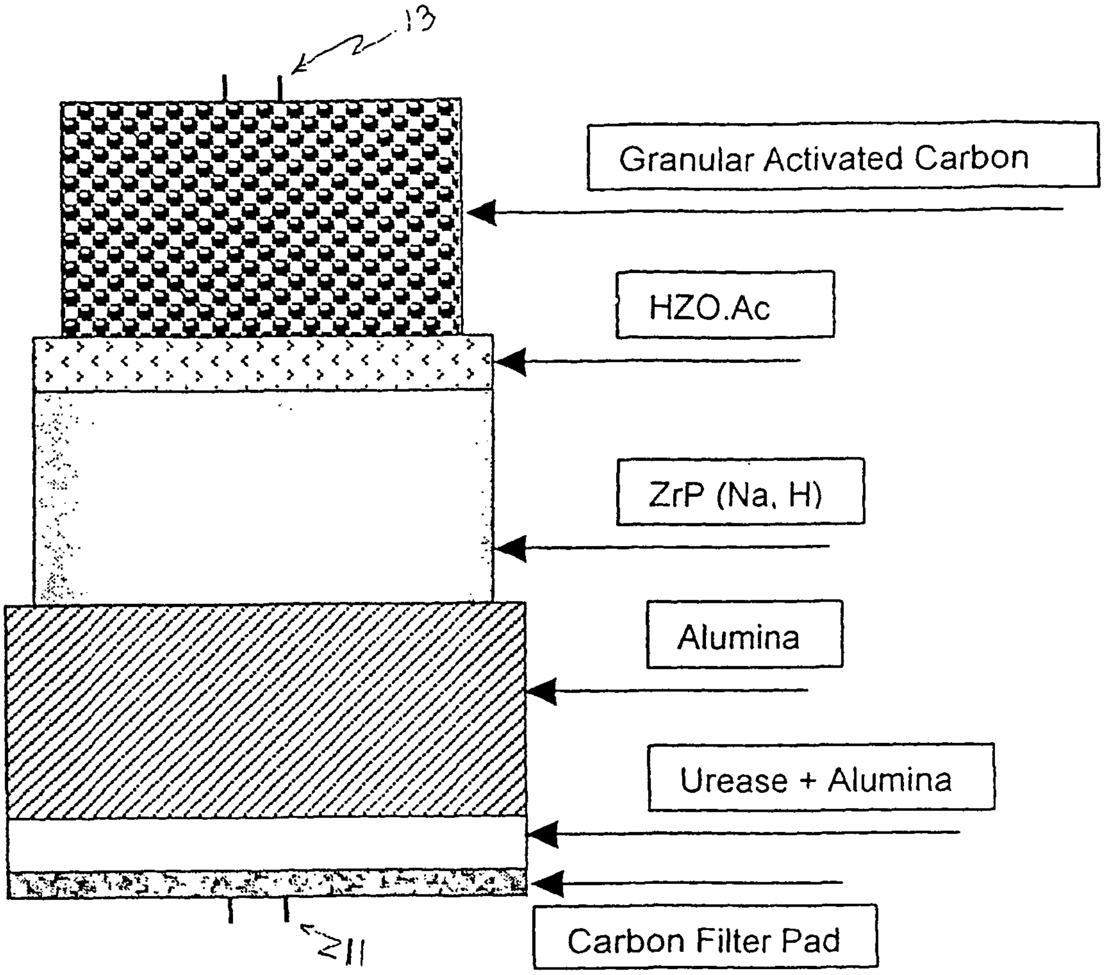
13
Granular Activated Carbon
HZO.Ac
ZrP (Na, H)
Alumina
Urease + Alumina
Carbon Filter Pad
211

Figure 1

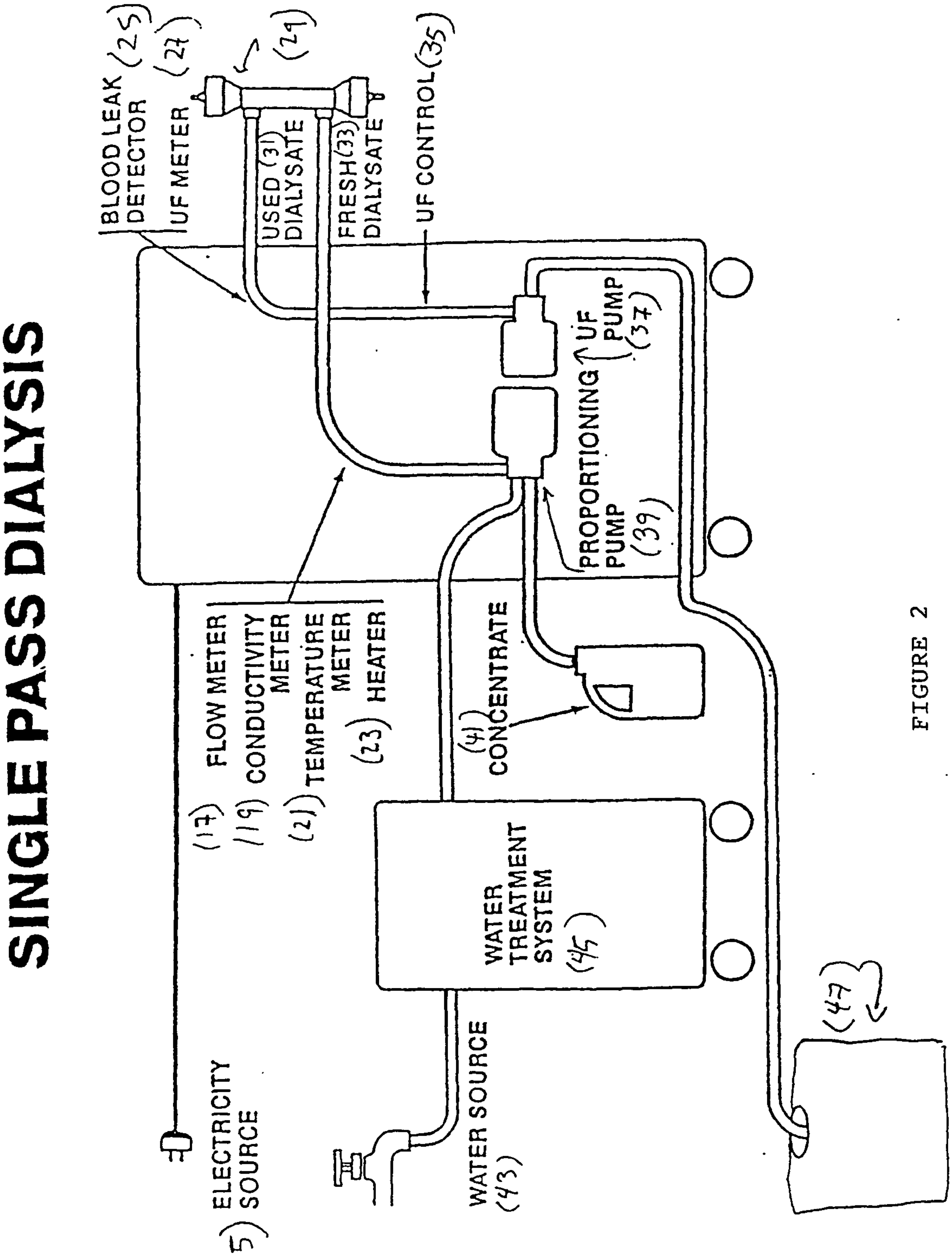
SINGLE PASS DIALYSIS
BLOOD LEAK DETECTOR (25)
UF METER (27)
(29)
USED (31) DIALYSATE
FRESH (33) DIALYSATE
UF CONTROL (35)
UF PUMP (37)
PROPORTIONING PUMP (39)
(17) FLOW METER
(19) CONDUCTIVITY METER
(21) TEMPERATURE METER
(23) HEATER
(41) CONCENTRATE
WATER TREATMENT SYSTEM (45)
(47)
(15) ELECTRICITY SOURCE
WATER SOURCE (43)

FIGURE 2

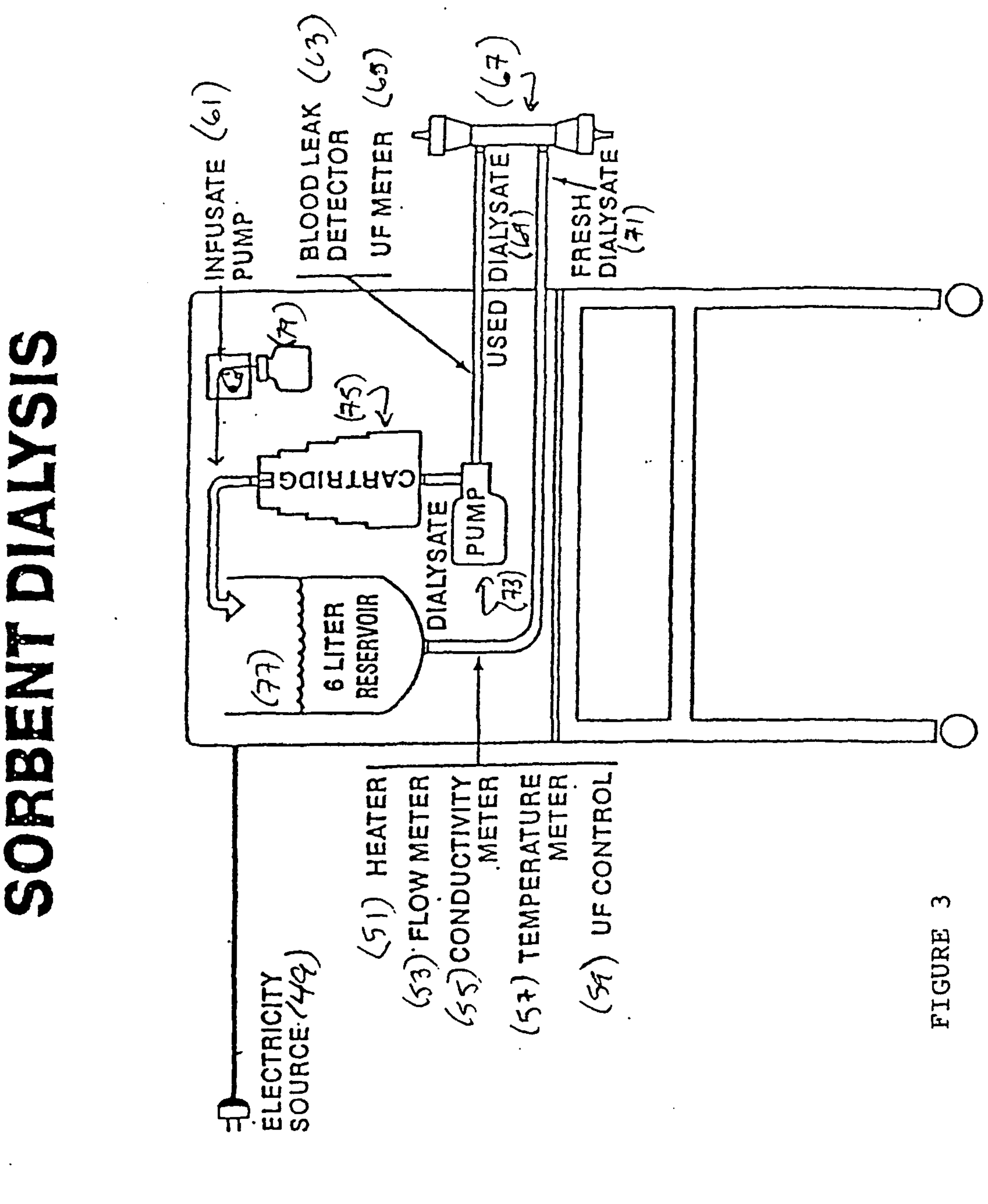
SORBENT DIALYSIS
INFUSATE PUMP (61)
BLOOD LEAK DETECTOR (63)
UF METER (65)
(67)
USED DIALYSATE (69)
FRESH DIALYSATE (71)
(79)
(75)
CARTRIDGE
PUMP
DIALYSATE (73)
DIALYSATE
6 LITER RESERVOIR
(77)
ELECTRICITY SOURCE (49)
(51) HEATER
(53) FLOW METER
(55) CONDUCTIVITY METER
(57) TEMPERATURE METER
(59) UF CONTROL

FIGURE 3

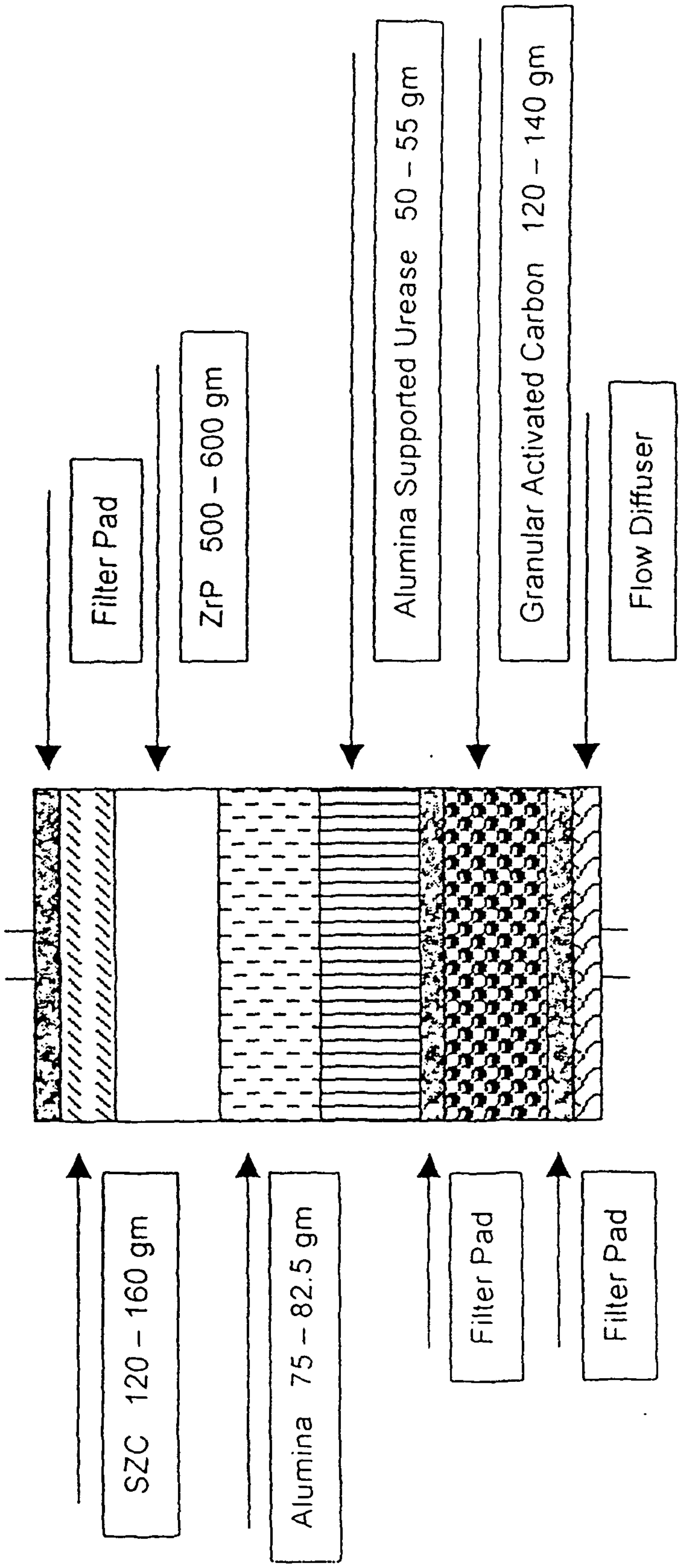
Filter Pad
ZrP 500 – 600 gm
Alumina Supported Urease 50 – 55 gm
Granular Activated Carbon 120 – 140 gm
Flow Diffuser
SZC 120 – 160 gm
Alumina 75 – 82.5 gm
Filter Pad
Filter Pad

FIGURE 4

Figure 5

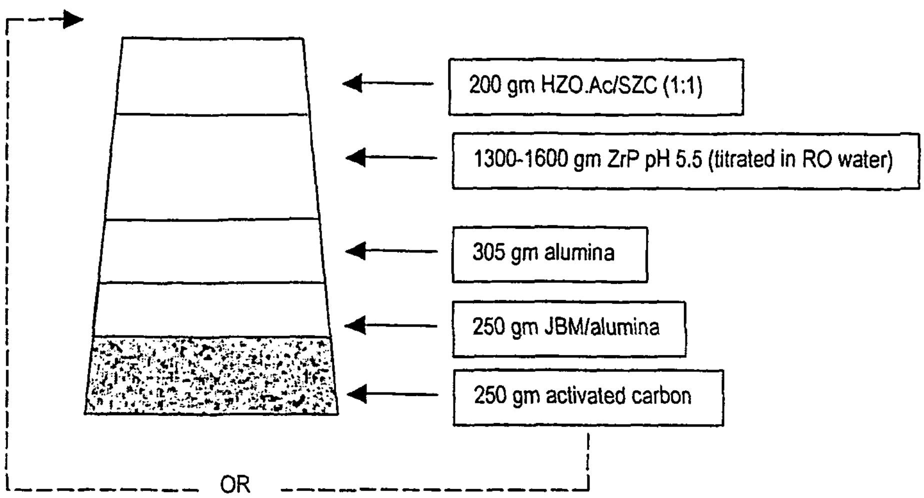


Figure 6

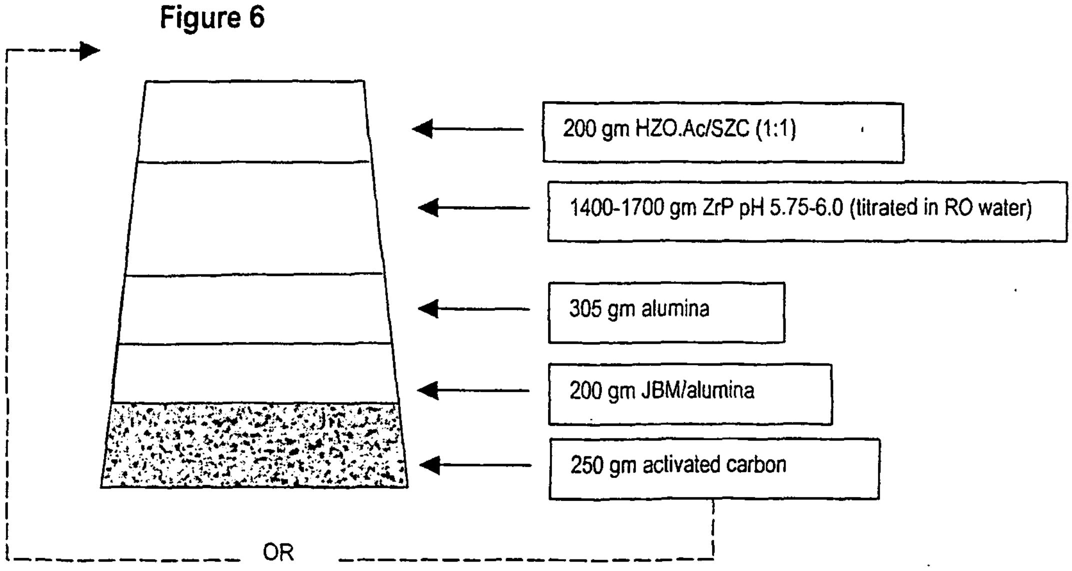

Figure 7

Fluid Regeneration Test System

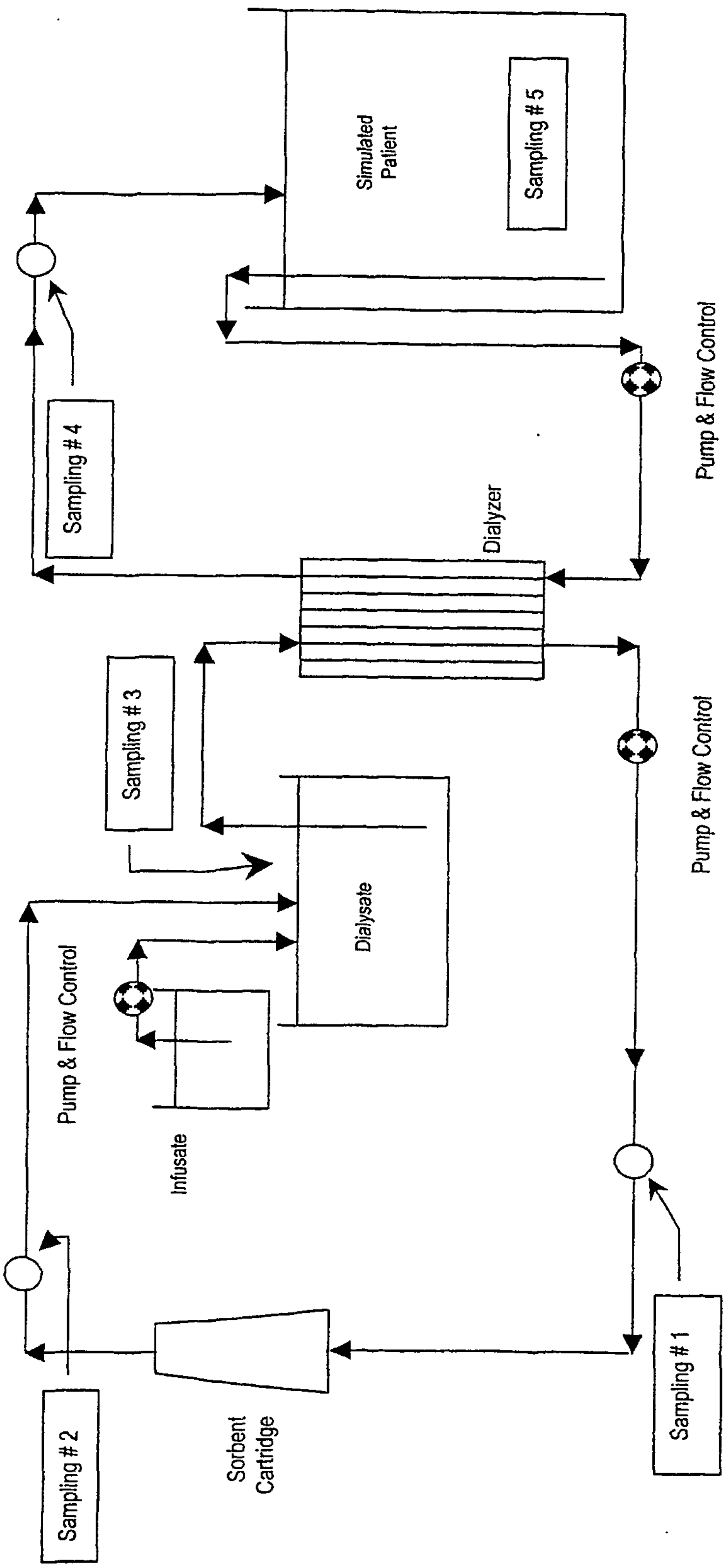

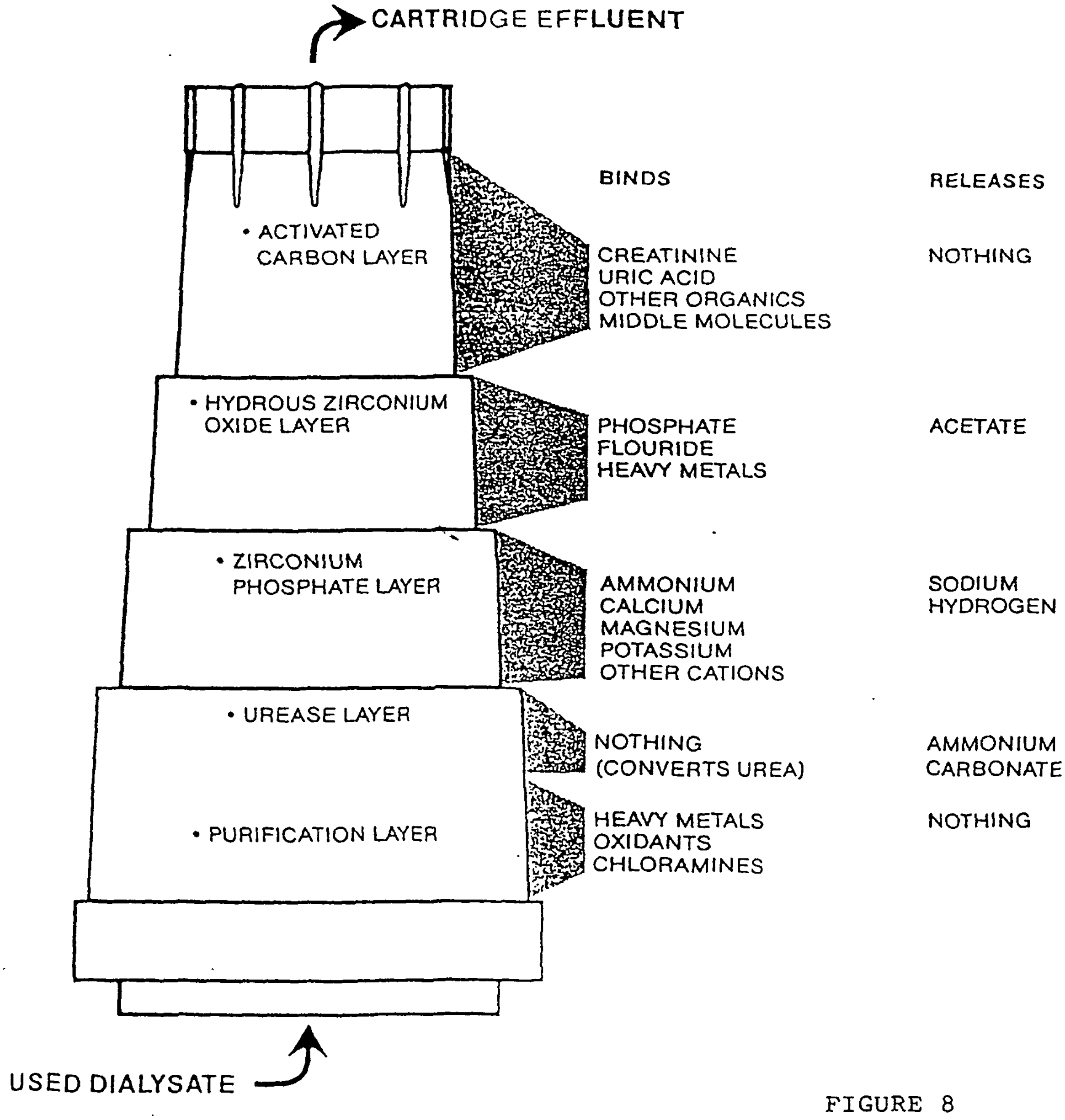
CARTRIDGE EFFLUENT
BINDS
RELEASES
• ACTIVATED
CARBON LAYER
CREATININE
URIC ACID
OTHER ORGANICS
MIDDLE MOLECULES
NOTHING
• HYDROUS ZIRCONIUM
OXIDE LAYER
PHOSPHATE
FLOURIDE
HEAVY METALS
ACETATE
• ZIRCONIUM
PHOSPHATE LAYER
AMMONIUM
CALCIUM
MAGNESIUM
POTASSIUM
OTHER CATIONS
SODIUM
HYDROGEN
• UREASE LAYER
NOTHING
(CONVERTS UREA)
AMMONIUM
CARBONATE
• PURIFICATION LAYER
HEAVY METALS
OXIDANTS
CHLORAMINES
NOTHING
USED DIALYSATE

FIGURE 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 59046964 A
- US 282578 A
- US 3669878 A
- US 3669880 A
- US 3697410 A
- US 3697418 A
- US 3703959 A
- US 3850835 A
- US 3989622 A
- US 3989625 A
- US 4025608 A
- US 4213859 A
- US 4256718 A
- US 4360507 A
- US 4460555 A
- US 4484599 A
- US 4495129 A
- US 4558996 A
- US 6309673 B
- US 6306836 B
- US 6196992 B
- US 6117122 B
- US 6074359 B
- US 6017942 B
- US 5984891 B
- US 5955450 B
- US 5938634 B
- US 5782796 B
- US 5631025 B
- US 5597805 B
- US 4560472 B
- US 6299769 B
- US 6284131 B
- US 6146536 B
- US 5968966 B
- US 5704915 B
- US 5824213 B
- US 5641405 B
- US 4738668 B
- US 6293921 B
- US 6284139 B
- US 6274103 B
- US 5980481 B
- US 5498338 B

### Non-patent literature cited in the description

- Guide to Custom Dialysis. *Product No. 306100-005,* September 1993, 1-54
- Sorbent Dialysis Primer. Product No. 306100-006. Cobe Renal Care, Inc, September 1993, 1-51